# FASCICULE DE BREVET EUROPEEN

(11) **EP 2 107 055 B1**
(45) Date de publication et mention de la délivrance du brevet: **16.03.2011**
(21) Numéro de dépôt: 09290237.8
(22) Date de dépôt: 31.03.2009
(51) Int. Cl.: C07D 311/30, C07D 407/12, A61K 31/352, A61K 31/7012, C07H 15/26, A61P 9/00, A61P 9/10, A61P 9/12

(54) **Nouveaux dérivés de diosmétine, leur procédé de préparation et les compositions pharmaceutiques qui les contiennent**
Diosmetin-Derivate, Verfahren zu deren Herstellung und diese enthaltende pharmazeutische Zusammensetzungen
Diosmetin derivatives, process for their preparation and pharmaceutical compositions containing them

(30) Priorité: 01.04.2008 FR 0801779
(43) Date de publication de la demande: 07.10.2009
(73) Titulaire: Les Laboratoires Servier, 92284 Suresnes Cedex (FR)
(72) Inventeur: Wierzbicki, Michel, 78620 L'Etang la Ville (FR); Boussard, Marie Françoise, 78124 Mareil sur Mauldre (FR); Verbeuren, Tony, 78540 Vernouillet (FR); Sansilvestri-Morel, Patricia, 92160 Antony (FR); Rupin, Alain, 37510 Savonnières (FR); Paysant, Jérôme, 92380 Gardes (FR); Lefoulon, François, 45000 Orléans (FR)
(74) Mandataire: Giudicelli, Cathy

(56) Documents cités:
- EP-A- 0 709 383
- WO-A-2006/092490
- US-A- 5 792 789

## Description

La présente invention a pour objet de nouveaux dérivés de diosmétine, leur procédé de préparation et les compositions pharmaceutiques qui les contiennent.

Des dérivés de diosmétine, ainsi que leur activité dans le traitement de l'insuffisance veineuse, ont été décrits dans le brevet EP 0 709 383.

Les composés de l'invention sont des inhibiteurs des molécules d'adhésion, des inhibiteurs de la NADPH oxydase, et des antiagrégants plaquettaires.

Les propriétés d'inhibition de l'adhésion leucocytaire et de la NADPH oxydase sont importantes dans le traitement de la maladie veineuse chronique, puisque dans cette pathologie, une inflammation du réseau microcirculatoire des membres inférieurs impliquant des infiltrations leucocytaires a été largement décrite (Verbeuren TJ, Bouskela E, Cohen RA et al, Regulation of adhesion molecules : a new target for the treatment of chronic venous insufficiency, 2000, Microcirculation, 7,S41-S48).

La propriété d'inhibition de l'agrégation plaquettaire démontre un potentiel anti-thrombotique des composés de l'invention, non seulement dans la prévention et le traitement des thromboses veineuses et artérielles, mais également dans le traitement de la maladie veineuse chronique, où les plaquettes peuvent être activées par des médiateurs inflammatoires, ou chez des patients présentant un syndrome post-thrombotique.

La présence d'une microangiopathie capillaire-veinulaire a été mise en évidence dans les maladies veineuses chroniques. Cette microangiopathie est la conséquence de l'hypertension veineuse et entraîne des troubles de filtration capillaire-veinulaire (hyperperméabilité) et donc des micro-oedèmes (Barbier et al., Microcirculation and rheology, 1994, Presse med. 23, 213-224). De nombreuses études ont montré l'implication de l'activation des cellules endothéliales dans l'hypertension veineuse associée à une élévation des taux circulants de molécules d'adhésion (Saharay M, Shields DA, Georgiannos SN et al, Endothelial activation in patients with chronic venous disease, 1998, Eur J Vasc Surg, 15, 342-349 ; Verbeuren TJ, Bouskela E, Cohen RA et al, Regulation of adhesion molecules : a new target for the treatment of chronic venous insufficiency, 2000, Microcirculation, 7,S41-S48).
Les composés de la présente invention possèdent non seulement une activité anti-inflammatoire mais aussi une activité d'anti-hyperperméabilité.

D'autre part, une augmentation de radicaux libres et donc une activation de la NADPH oxydase a été mise en évidence dans les maladies veineuses chroniques. Ce stress oxydant serait lié à l'activation des cellules endothéliales et l'infiltration leucocytaire (Glowinski J and Glowinski S, Generation of reactive oxygen metabolites by the varicose vein wall, 2002, Eur. J. Vasc. Endovasc. Surg ., 23, 5550-555).
L'activation des cellules endothéliales et l'induction de molécules d'adhésion et de la NADPH oxydase est démontrée dans plusieurs pathologies vasculaires (Bedard K and Krause KH, The NOX family of ROS-generating oxidases : Physiology and pathophysiology, 2007, Physiol. Rev. 87, 245-313).

Ainsi, les composés de la présente invention sont utilisables dans la prévention ou le traitement des maladies veineuses, notamment la maladie veineuse chronique à tous ses stades (douleurs, télangectasie, veines variqueuses, oedèmes, troubles trophiques, ulcères), ainsi que dans la prévention ou le traitement du syndrome post-thrombotique, des complications vasculaires liées au diabète, de l'hypertension, de l'athérosclérose, de l'inflammation, du syndrome métabolique lié à l'obésité, des complications vasculaires liées à l'obésité, de l'angine de poitrine, des artérites des membres inférieurs ou des accidents vasculaires cérébraux, de la cicatrisation des plaies chroniques incluant les ulcères de jambe à prédominance veineuse ou mixtes et le pied diabétique, dans le traitement ou la prévention des crises hémorroïdaires, dans le traitement ou la prévention des escarres et dans le traitement de la sclérose en plaque.

La présente invention concerne plus spécialement les composés de formule (I) : 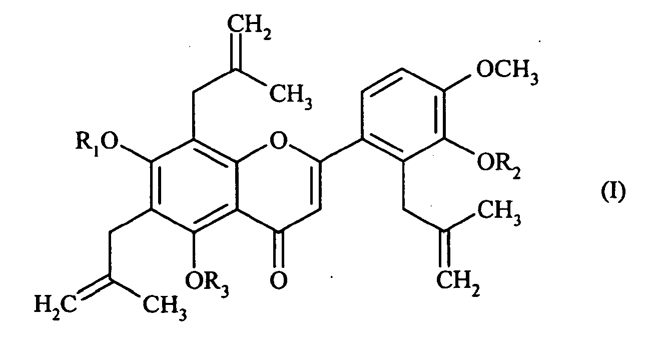 dans laquelle R₁, R₂ et R₃, identiques ou différents, représentent chacun un atome d'hydrogène ou le groupement de formule (A) :

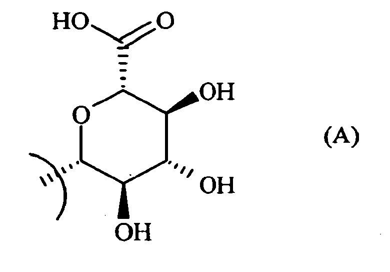

Les composés pour lesquels l'un au moins des R₁, R₂ et R₃ représente un groupement (A), sont des métabolites du composé de formule (Ia) pour lequel R₁, R₂ et R₃ représentent chacun un atome d'hydrogène.

La présente invention a également pour objet le procédé de préparation des composés de formule (I), à partir de la diosmétine de formule (II) : 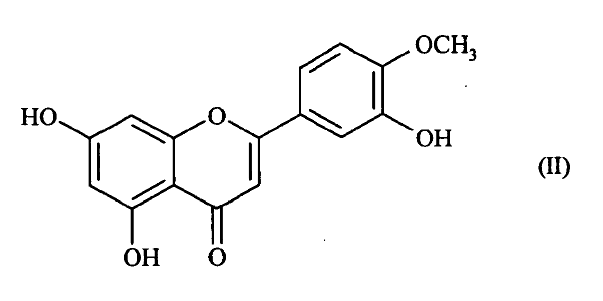 que l'on fait réagir avec le bromure de méthallyle, pour conduire au composé de formule (III) : 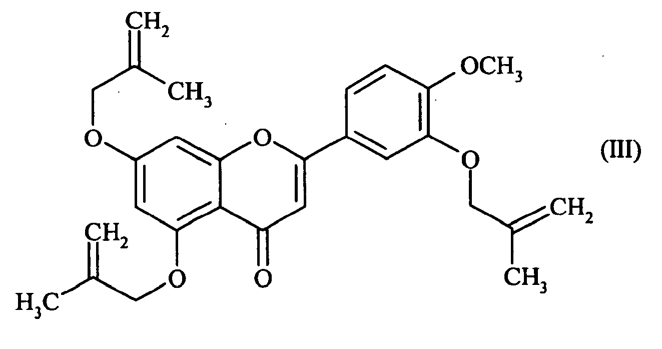 que l'on chauffe pour conduire au composé de formule (Ia), cas particulier des composés de formule (I) pour lequel R₁, R₂ et R₃ représentent chacun un atome d'hydrogène : 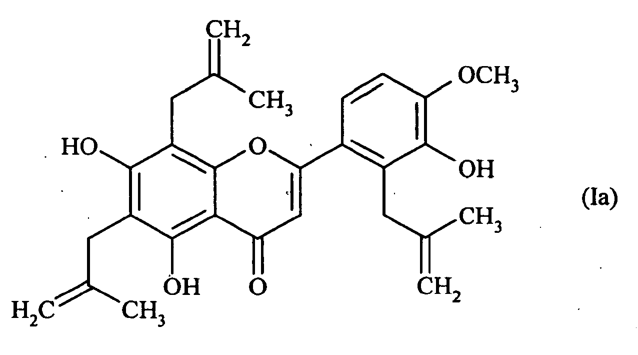 que l'on fait réagir, lorsque l'on souhaite accéder aux autres composés de formule (I), avec le composé de formule (IV) : 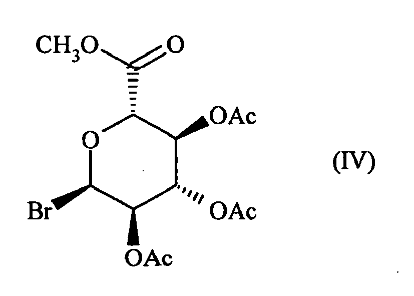 dans laquelle Ac représente le groupement acétyle,
pour conduire, après déprotection de la fonction acide et des fonctions alcool du groupement (A), aux composés de formule (I) pour lesquels au moins l'un des R₁, R₂ et R₃ est différent de H.

Lorsque les composés de formule (I) sont obtenus en mélange, on peut les séparer, par exemple par chromatographie HPLC préparative.

Le composé de formule (Ib), pour lequel R₁ et R₃ représentent chacun un atome d'hydrogène et R₂ représente un groupement de formule (A), peut également être obtenu par acétylation du composé de formule (Ia), pour conduire au composé de formule (V) : 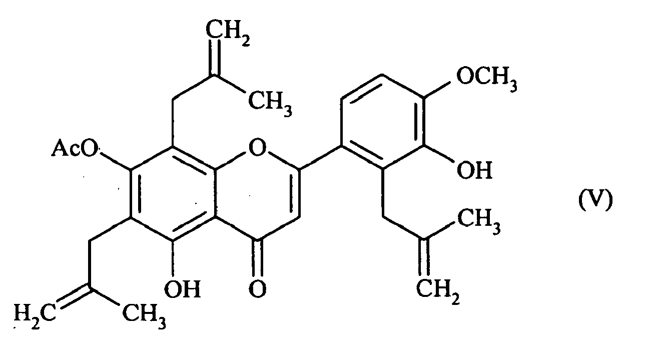 dans laquelle Ac représente le groupement acétyle,
que l'on met en réaction avec le composé de formule (IV), pour conduire au composé de formule (VI) : 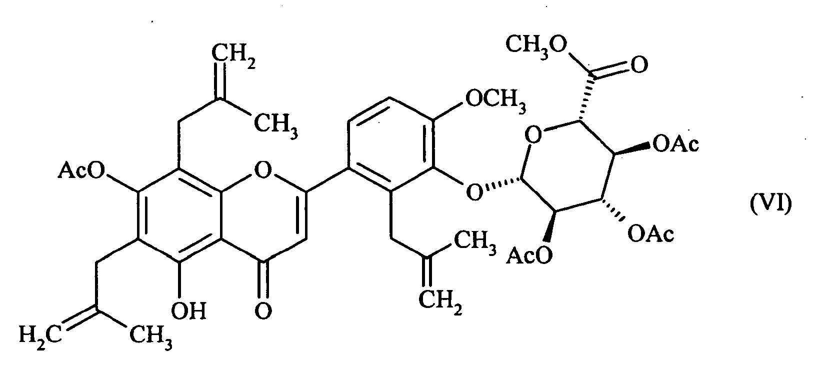 dans laquelle Ac représente le groupement acétyle,
dont on déprotège la fonction acide et les fonctions alcool et phénol, pour conduire au composé de formule (Ib).

Les composés de l'invention sont des inhibiteurs des molécules d'adhésion et de la NADPH oxydase, et des antiagrégants plaquettaires.

A ce titre, ils sont utiles dans la prévention ou le traitement des maladies veineuses, notamment la maladie veineuse chronique à tous ses stades (douleurs, télangectasie, veines variqueuses, oedèmes, troubles trophiques, ulcères), ainsi que dans la prévention ou le traitement du syndrome post-thrombotique, des complications vasculaires liées au diabète, de l'hypertension, de l'athérosclérose, de l'inflammation, du syndrome métabolique lié à l'obésité, des complications vasculaires liées à l'obésité, de l'angine de poitrine, des artérites des membres inférieurs ou des accidents vasculaires cérébraux, de la cicatrisation des plaies chroniques incluant les ulcères de jambe à prédominance veineuse ou mixtes et le pied diabétique, dans le traitement ou la prévention des crises hémorroïdaires, dans le traitement ou la prévention des escarres et dans le traitement de la sclérose en plaque.

La présente invention a également pour objet les compositions pharmaceutiques contenant comme principe actif un composé de formule (I), en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 1000 mg en une ou plusieurs prises par jour.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

### ABREVIATIONS

DMSO : diméthylsulfoxyde
NADPH : forme réduite du Nicotinamide Adénine Dinucléotide Phosphate
HPLC : Chromatographie en Phase Liquide à Haute Performance

### EXEMPLE 1 : 6,8,2'-Tris-(isobut-2-en-1-yl) diosmétine

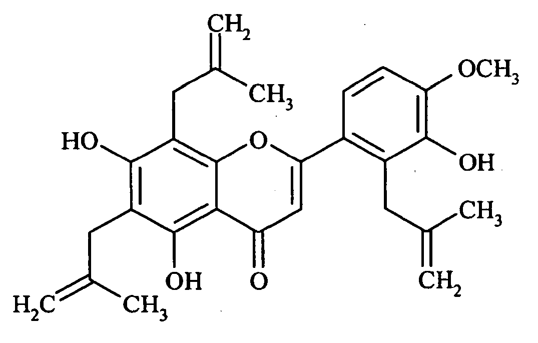

### Stade A : 2-{4-Méthoxy-3-[(isobut-2-en-1-yl)oxy)phényl)-5,7-bis-[(isobut-2-en-1-yl)oxy)-4H-chromen-4-one

A 30 g de diosmétine sont ajoutés 69,3 g de carbonate de potassium et 450 ml d'acétone. Le mélange est chauffé au reflux pendant 4h30, puis ramené à température ambiante, puis 54 g de bromure de méthallyle sont ajoutés. Le mélange réactionnel est ensuite chauffé à reflux pendant la nuit, puis ramené à température ambiante et filtré. Le gâteau est rincé à l'acétone puis le filtrat est évaporé pour conduire à un résidu qui est recristallisé dans le toluène pour conduire au produit du titre.

### Stade B : 6,8,2'-Tris-(isobut-2-en-1-yl) diosmétine

A 10 g du composé obtenu au stade précédent sont ajoutés 120 ml de N,N-diméthylaniline, puis le mélange est chauffé au reflux pendant 1h. Le solvant est ensuite évaporé sous pression réduite et le résidu obtenu est recristallisé dans l'isopropanol pour conduire au produit du titre. *Point de fusion* : 141°C.

### EXEMPLE 2 : Acide (5-hydroxy-2-[3-hydroxy-4-méthoxy-2-(isobut-2-en-1-yl)phényl]-6,8-bis(isobut-2-en-1-yl)-4-oxo-4H-chromen-7-yl)bêta-D-glucuronique

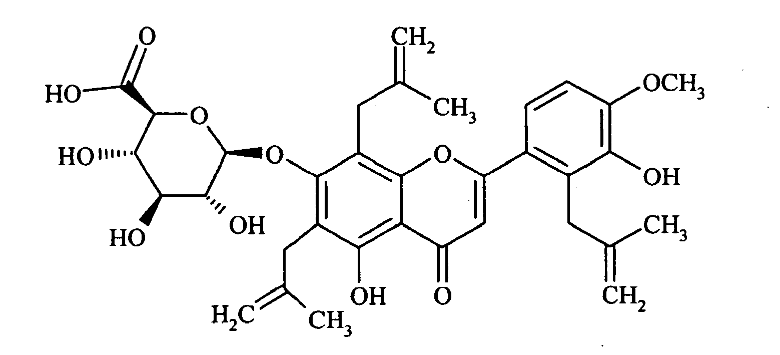

### Stade A : 5-Hydroxy-2-[3-hydroxy-4-méthoxy-2-(isobut-2-en-1-yl)phényl]-6,8-bis(isobut-2-en-1-yl)-4-oxo-4H-chromen-7-yl 2,3,4-tris-O-acétyl-bêta-D-glucuronate de méthyle

Le composé du titre est obtenu par réaction du composé de l'Exemple 1 (250 mg) avec le composé de formule (IV) (429 mg), en catalyse par transfert de phase, selon le procédé décrit dans la publication Synth Commun 1999, 29(16), 2775-2781.

### Stade B : Acide (5-hydroxy-2-{3-hydroxy-4-méthoxy-2-(isobut-2-en-l-yl)phény/}-6,8-bis(isobut-2-en-1-yl)-4-oxo-4H-chromen-7-yl)bêta-D-glucuronique

Le composé obtenu au stade A est mis en solution dans du méthanol, puis de la soude est ajoutée. Le milieu est porté au reflux pendant 1h30, puis neutralisé avec une solution d'acide chlorhydrique 2N avant d'être évaporé à sec pour conduire au produit du titre.

### EXEMPLE 3 : Acide 3-[5,7-dihydroxy-6,8-bis(isobut-2-en-1-yl)-4-oxo-4H-chromen-2-yl]6-méthoxy-2-(isobut-2-en-1-yl)phényl bêta-D-glucuronique

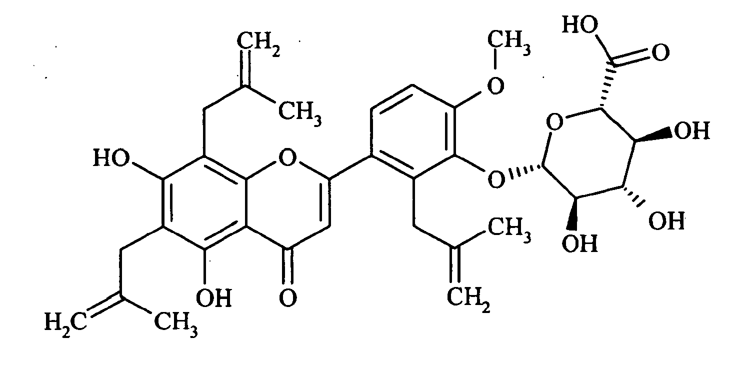

### Stade A : Acétate de5-hydroxy-2-[3-hydroxy-4-méthoxy-2-(isobut-2-en-l-yl)phény/]-6,8-bis(isobut-2-en-1-yl)-4-oxo-4H-chromen-7-yle

Le composé de l'Exemple 1 (3 g) est mis en solution dans la pyridine, puis de l'anhydride acétique (0,61 ml) est ajouté à température ambiante. Le milieu réactionnel est ensuite agité pendant 16h puis évaporé à sec. Le résidu est repris par de l'eau glacée puis extrait au dichlorométhane, séché, filtré et évaporé. Le produit brut ainsi obtenu est purifié sur gel de silice puis par HPLC préparative en phase inverse, pour conduire au produit du titre.

### Stade B : 3-[7-(Acétyloxy)-5-hydroxy-6,8-bis(isobut-2-en-1yl)-4-oxo-4H chromen-2 yl]-6-méthoxy-2-(isobut-2-en-1-yl)phényl 2,3,4-tris-O-acétyl-bêta-D-glucuronate de méthyle

Le composé du titre est obtenu selon le procédé du stade A de l'Exemple 2, à partir du composé obtenu au stade précédent.

### Stade C : Acide 3-[5,7-dihydroxy-6,8-bis(isobut-2-en-1-yl)-4-oxo-4H-chromen-2-yl]6-méthoxy-2-(isobut-2-en-1-yl)phényl bêta-D-glucuronique

Le composé du titre est obtenu selon le procédé du stade B de l'Exemple 2, à partir du composé obtenu au stade précédent.

### ETUDE PHARMACOLOGIQUE

Dans les exemples suivants, le terme "composé de référence" se rapporte à l'Exemple 69 de EP 0 709 383.

### EXEMPLE 4 : Inhibition de l'agrégation des plaquettes in vitro

Sur des lapins néo-zélandais anesthésiés, un prélèvement de sang sur citrate 0,109 M est réalisé au niveau de la carotide. Le plasma riche en plaquettes est récupéré par centrifugation. Les plaquettes sont ensuite lavées par centrifugation.

Les plaquettes lavées sont remises en suspension dans un tampon tyrode. La suspension plaquettaire est mise dans une cuve puis dans un agrégomètre à 37°C sous agitation en présence du composé de l'Exemple 1 (30 µM) ou du composé de référence (30 µM) dilués dans le même solvant (0,1% DMSO). Après 2 minutes, l'agrégation est induite par le collagène (4 µg/ml), la réponse est ensuite enregistrée pendant 6 minutes. La quantification de l'agrégation plaquettaire se fait par turbidimétrie, c'est à dire le pourcentage de lumière transmise au travers de la suspension plaquettaire par rapport à une cuve contenant du tyrode et une cuve contenant le solvant (0,1% DMSO).

L'efficacité antiagrégante des dérivés de la diosmétine selon l'invention et en particulier du composé de l'Exemple 1 et du composé de référence est évaluée en fonction du pourcentage d'inhibition de l'agrégation des plaquettes, cette activité sera d'autant plus importante que le pourcentage d'inhibition sera grand. Le composé de l'Exemple 1 (30 µM) provoque une inhibition de 36,6±9,9% alors que le composé de référence ne provoque pas d'effet significatif (4,1± 1,8%) ; (P<0,01 composé de l'Exemple 1 par rapport au composé de référence, test t de Student, n=7).

Ce test démontre l'activité inhibitrice de l'agrégation plaquettaire et donc le potentiel anti thrombotique du composé de l'Exemple 1.

### EXEMPLE 5 : Inhibition de l'adhésion des leucocytes in vivo

Trois groupes de 3 hamsters, pesant 90 à 110g, sont utilisés dans cette étude. Trente minutes avant anesthésie, les hamsters sont traités oralement avec une dose unique de placebo (gomme arabique 10%), de composé de l'Exemple 1 (3 mg/kg) ou de composé de référence (3 mg/kg). Les animaux sont anesthésiés au pentobarbital 50 mg/kg par administration intra-péritonéale. Les hamsters sont placés sous microscope et la bajoue est isolée et immergée dans une solution de perfusion (NaCl 110,0 mM, KCl 4,7 mM, CaCl₂ 2,0 mM, MgSO₄ 1,2 mM, NaHCO₃ 18,0 mM, Hepes 15,39 mM et Hepes Na⁺-salt 14,61 mM), (Duling, The preparation and use of the hamster cheek pouch for studies of the microcirculation, 1973, Microvasc. Res. 5 : 423-429 ; Svensjö et al., The hamster cheek pouch preparation as a model for studies of macromolecular permeability of the microvasculature, 1978, Uppsala J. Med. Sci. 83 : 71-79).
Une ischémie locale est réalisée à l'aide d'un tube en latex monté à l'entrée de la bajoue. La pression intratubulaire du tube est augmentée jusqu'à 200-220 mm Hg à l'aide d'une seringue calibrée. Cette occlusion totale est réalisée pendant 30 minutes, puis une reperfusion est effectuée pendant 45 minutes. L'adhésion des leucocytes aux cellules endothéliales dans les veinules post capillaires est quantifiée dans un champ de 6 mm² juste après le début de l'ischémie (défini comme 100%) puis à différents temps après reperfusion (0, 15, 30 et 45 minutes).

Le modèle d'adhésion leucocytaire induite par ischémie reperfusion dans la bajoue de hamster permet de vérifier l'efficacité des dérivés de la diosmétine selon l'invention en tant qu'agents anti-adhésifs et en particulier du composé de l'Exemple 1 et du composé de référence.

L'activité du composé de l'Exemple 1 et du composé de référence est évaluée en fonction du nombre de leucocytes adhérés aux cellules endothéliales pour un champ de 6 mm² après ischémie/reperfusion, cette activité sera d'autant plus importante que le nombre de leucocytes sera faible et donc que le pourcentage de leucocytes adhérés par rapport au nombre de leucocytes adhérés après ischémie sera faible.

**Tableau 1 : Effet du traitement oral des hamsters avec le composé de l'Exemple 1 ou le composé de référence sur le nombre de leucocytes adhérés aux cellules endothéliales dans les veinules postcapillaires de la bajoue après ischémie (nombre de leucocytes considéré comme 100%) et après 0, 15, 30 et 45 minutes de reperfusion.**

| **Temps après reperfusion (minutes)** | **Placebo** | **Composé de l'Exemple 1** | **Composé de référence** |
|---|---|---|---|
| 0 | 165,8±12,8% | 81,7±19,1% ** | 129,3±10,9% |
| 15 | 211,8±11,6% | 100±13,7% *** | 138,0±14,4% ** |
| 30 | 210,5±16,6% | 91,0±18.7% *** | 121,0±18,0% ** |
| 45 | 166,3±11,1% | 109,3±23,0% | 133,7±42,4% |

| | | | |
|---|---|---|---|
| ** : p<0.01 ; *** : p<0.001 par rapport au traitement Placebo, ANOVA 2 facteurs (temps et traitement) suivi d'un test de Bonferroni (n=3). | | | |

Le composé de l'Exemple 1 permet de diminuer nettement et significativement le nombre de leucocytes adhérés aux cellules endothéliales après ischémie/reperfusion par rapport au Placebo. L'activité du composé de l'Exemple 1 est plus puissante que celle du composé de référence.

Ce test démontre l'activité inhibitrice de l'adhésion leucocytaire du composé de l'Exemple 1 et donc un potentiel pour le traitement de la maladie veineuse ainsi que des maladies vasculaires artérielles telles que l'athérosclérose ou les complications vasculaires liées au diabète.

### EXEMPLE 6 : Inhibition de l'expression de Vascular Cell Adhesion Molecule 1 (VCAM-1) in vivo

Quatre groupes de 8 souris déficientes en protéine apolipoprotéine E (ApoE^{-/-}, développant spontanément des plaques d'athérome dans les aortes) sont utilisés dans cette étude. A l'âge de 9 semaines, les souris sont rendues diabétiques par 5 injections intra-péritonéales de 100 mg/kg de streptozotocine sur 5 jours. A la dixième semaine, les animaux sont partagés en quatre groupes : groupe contrôle composé de l'Exemple 1, groupe traité composé de l'Exemple 1 (130 mg/kg /jour dans la nourriture pendant 6 semaines), groupe contrôle composé de référence, groupe traité composé de référence (130 mg/kg /jour dans la nourriture pendant 6 semaines). Les souris sont sacrifiées à la quinzième semaine après anesthésie à l'isoflurane. Les aortes sont prélevées, disséquées et congelées dans l'azote liquide.
Les aortes sont cryobroyées, et les ARN totaux sont extraits à l'aide du kit RNeasy® micro (Qiagen). La transcription inverse est ensuite réalisée à partir de 1µg d'ARN total à l'aide du kit Superscript^{™} III first-strand cDNA synthesis (Invitrogen). L'expression de VCAM-1 est quantifiée par PCR en temps réel et normalisée par rapport à 3 gènes de référence : β actine, hypoxanthine-guanine phosphoribosyl transferase (HPRT) et glyceraldehyde phosphate deshydrogenase (GAPDH). Le kit IQ^{™} SYBR® Green supermix (Biorad) est utilisé, avec 2µl d'ADNc et 150 nM de chaque amorce. Les échantillons sont dénaturés 5 minutes à 95°C et amplifiés durant 40 cycles selon le protocole suivant : dénaturation pendant 20 secondes à 95°C et hybridation et élongation pendant 1 minute à 54°C pour VCAM-1, β actine et HPRT, 56°C pour GAPDH. Le cycle seuil (défini comme celui pour lequel la fluorescence est considérée comme significativement plus élevée que le bruit de fond) pour VCAM-1 des animaux non traités est normalisé par rapport aux gènes de référence (et considéré comme 100%) puis comparé à celui des animaux traités.
Les amorces spécifiques utilisées sont les suivantes :
VCAM-1 : 5'-AGA GCA GAC TTT CTA TTT CAC-3' (sens) et 5'-CCA TCT TCA CAG GCA TTT C-3' (antisens) ; β actine : 5'-AAG ACC TCT ATG CCA ACA CAG-3' (sens) et 5'-AGC CAC CGA TCC ACA CAG-3' (antisens) ; HPRT : 5'-AGC TAC TGT AAT GAT CAG TCA ACG-3' (antisens) ; GAPDH : 5'-GCC TTC CGT GTT CCT ACC C-3' (sens) et 5'-TGC CTG CTT CAC CAC CTT-3' (antisens).

Le modèle d'induction du diabète chez des souris déficientes en ApoE permet de vérifier l'efficacité des dérivés de la diosmétine selon l'invention en tant qu'agents anti-adhésifs.

L'activité du composé de l'Exemple 1 et du composé de référence est évaluée en fonction du niveau d'expression de VCAM-1 dans l'aorte comparé aux animaux non traités. Cette activité sera d'autant plus importante que le niveau d'expression de VCAM-1 sera faible. Les souris traitées par le composé de l'Exemple 1 ont un niveau d'expression de VCAM-1 de 65,9±10.1% par rapport aux souris non traitées (P<0,01, test t de Student, n=8) alors que celles traitées avec le composé de référence ont un niveau d'expression de VCAM-1 de 83,0±6,6% (P<0,05, test t de Student, n=8).
Le composé de l'Exemple 1 permet de diminuer nettement et significativement l'expression de VCAM-1 dans l'aorte de souris diabétiques ApoE^{-/-} par rapport au groupe non traité. L'activité du composé de l'Exemple 1 est plus puissante que celle du composé de référence.
Ce test démontre les activités inhibitrices de l'expression des molécules d'adhésion du composé de l'Exemple 1 et donc un potentiel pour le traitement de la maladie veineuse, ainsi que dans les pathologies artérielles telles que les complications vasculaires liées au diabète, l'hypertension, l'athérosclérose, l'inflammation, le syndrome métabolique lié à l'obésité, les complications vasculaires liées à l'obésité, l'angine de poitrine, artérites des membres inférieurs, accidents vasculaires cérébraux.

### EXEMPLE 7 : Inhibition de l'activité de la NADPH oxydase in vitro

L'étude est réalisée sur des cellules endothéliales humaines HUVEC (*Human Umbilical Vein Endothelial Cells*, Clonetics Co). Les cellules sont cultivées dans un milieu EBM2 (*Endothelial Basal Medium*, Clonetics Co) supplémenté avec 2% SVF (*Sérum de Veau Foetal)* et EGM2 (*Endothelial Growth Medium*, Clonetics Co).
Les cellules sont incubées en présence de solvant (0,1% DMSO, contrôle composé de l'Exemple 1), de EBM2 (contrôle des composés des Exemples 2 et 3), du composé de l'Exemple 1 (100µM), du composé de l'Exemple 2 (100 µM) ou du composé de l'Exemple 3 (100 µM) durant 15 minutes puis activées par de l'angiotensine II (1µM) pendant 30 minutes pour activer la NADPH oxydase. Les cellules sont lavées dans l'EBM2 puis le substrat de la NADPH oxydase (NADPH, 200 µM) et la lucigénine (25 µM) sont ajoutées. La réduction de la lucigénine par les anions superoxydes produits par la NADPH oxydase est quantifiée au luminomètre. Le nombre de coups par seconde (cps) des groupes contrôles est comparé aux groupes traités. Les cps obtenus avec les groupes contrôles sont considérés comme 100% de l'activité NADPH oxydase.

Le modèle de mesure de l'activité NADPH oxydase endothéliale induite par l'angiotensine II permet de vérifier l'efficacité des dérivés de la diosmétine selon l'invention en tant qu'agents inhibiteurs de l'activité de la NADPH oxydase.
L'activité des composés des Exemples 1, 2 et 3 est évaluée en fonction du nombre de cps obtenu, cette activité sera d'autant plus importante que le nombre de cps sera faible.

**Tableau 3 : Effet du traitement des cellules endothéliales humaines avec les composés des Exemples 1, 2 et 3 sur l'activité de la NADPH oxydase après induction par de l'angiotensine II (activité du groupe contrôle sans produit considéré comme 100%).**

| Groupe | Activité de la NADPH oxydase (% / groupe contrôle) |
|---|---|
| Groupe contrôle composé de l'Exemple 1 | 100% |
| Groupe traité composé de l'Exemple 1 (100µM) | 38,16±3,13% *** |
| Groupe contrôle composés des Exemples 2 et 3 | 100% |
| Groupe traité composé de l'Exemple 2 (100µM) | 30,60±4,83% *** |
| Groupe traité composé de l'Exemple 3 (100µM) | 18,45+4,08% *** |

| | |
|---|---|
| *** : p<0,01 par rapport au groupe contrôle du composé de l'Exemple 1 ou des composés des Exemples 2 et 3, Test t de Student (n=3). | |

Les composés des Exemples 1, 2 et 3 permettent de diminuer nettement et significativement l'activité de la NADPH oxydase dans les cellules endothéliales humaines.

Ce test démontre les activités inhibitrices de l'activité NADPH oxydase vasculaire des composés des Exemples 1, 2 et 3 et donc un potentiel inhibiteur de radicaux libres dans la maladie veineuse ainsi que dans les pathologies artérielles telles que l'athérosclérose, l'hypertension, les complications vasculaires liées au diabète et les maladies ischémiques.

### EXEMPLE 8 : Composition pharmaceutique

Formule de préparation pour 1000 comprimés dosés à 10 mg :

| | |
|---|---|
| Composé de l'Exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 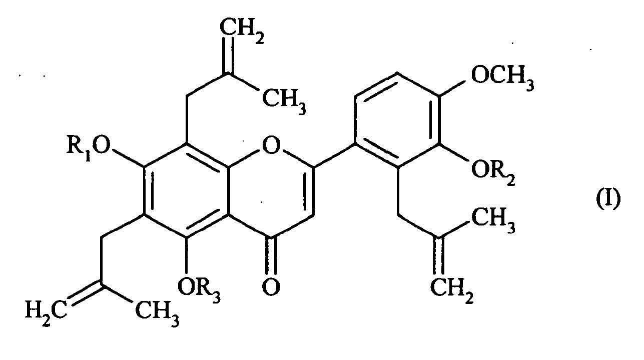 dans laquelle R₁, R₂ et R₃, identiques ou différents, représentent chacun un atome d'hydrogène ou le groupement de formule (A) :

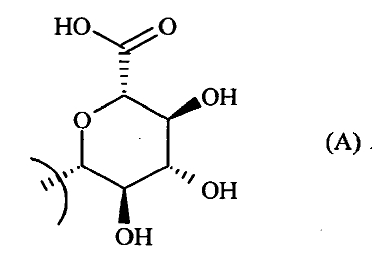

2. Composé de formule (I) selon la revendication 1, choisi parmi :
- la 6,8,2'-tris-(isobut-2-en-1-yl) diosmétine,
- l'acide (5-hydroxy-2-[3-hydroxy-4-méthoxy-2-(isobut-2-en-1-yl)phényl]-6,8-bis(isobut-2-en-1-yl)-4-oxo-4*H*-chromen-7-yl) bêta-D-glucuronique, et
- l'acide 3-[5,7-dihydroxy-6,8-bis(isobut-2-en-1-yl)-4-oxo-4*H*-chromen-2-yl]6-méthoxy-2-(isobut-2-en-1-yl)phényl bêta-D-glucuronique.

3. Procédé de synthèse des composés de formule (I) selon la revendication 1 à partir de la diosmétine de formule (II) : 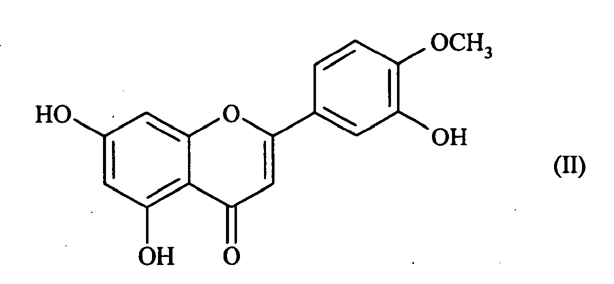 que l'on fait réagir avec le bromure de méthallyle, pour conduire au composé de formule (III) : 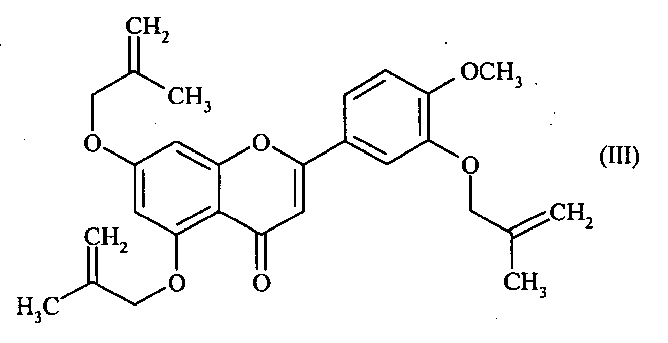 que l'on chauffe, pour conduire au composé de formule (Ia), cas particulier des composés de formule (I) pour lequel R₁, R₂ et R₃ représentent chacun un atome d'hydrogène : 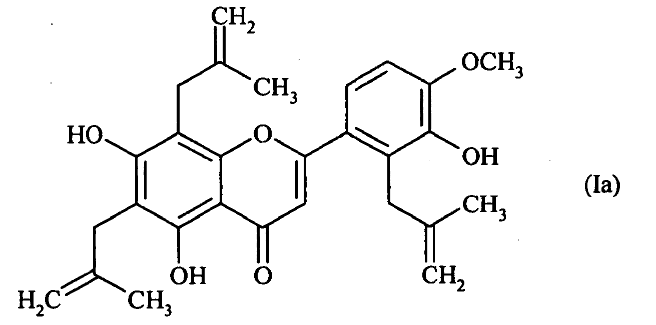 que l'on fait réagir, lorsque l'on souhaite accéder aux autres composés de formule (I), avec le composé de formule (IV) : 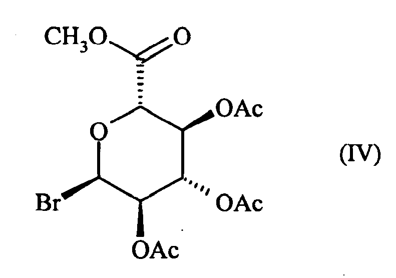 dans laquelle Ac représente le groupement acétyle,
pour conduire, après déprotection de la fonction acide et des fonctions alcool du groupement (A) tel que défini dans la revendication 1, aux composés de formule (I) pour lesquels au moins l'un des R₁, R₂ et R₃ est différent de H.

4. Composition pharmaceutique contenant comme principe actif un composé de formule (I) selon l'une quelconque des revendications 1 ou 2, en combinaison avec un ou plusieurs excipients ou véhicules inertes, non toxiques, pharmaceutiquement acceptables.

5. Utilisation d'un composé selon l'une quelconque des revendications 1 ou 2 pour la fabrication de médicaments utiles pour la prévention ou le traitement des maladies veineuses, pour la prévention ou le traitement du syndrome post-thrombotique, des complications vasculaires liées au diabète, de l'hypertension, de l'athérosclérose, de l'inflammation, du syndrome métabolique lié à l'obésité, des complications vasculaires liées à l'obésité, de l'angine de poitrine, des artérites des membres inférieurs ou des accidents vasculaires cérébraux, pour la cicatrisation des plaies chroniques incluant les ulcères de jambe à prédominance veineuse ou mixtes et le pied diabétique, pour le traitement ou la prévention des crises hémorroïdaires, pour le traitement ou la prévention des escarres et pour le traitement de la sclérose en plaque.

6. Utilisation d'un composé selon l'une quelconque des revendications 1 ou 2 pour la fabrication de médicaments utiles pour la prévention ou le traitement de la maladie veineuse chronique.

7. Composé selon l'une quelconque des revendications 1 ou 2 utile pour la prévention ou le traitement des maladies veineuses, pour la prévention ou le traitement du syndrome post-thrombotique, des complications vasculaires liées au diabète, de l'hypertension, de l'athérosclérose, de l'inflammation, du syndrome métabolique lié à l'obésité, des complications vasculaires liées à l'obésité, de l'angine de poitrine, des artérites des membres inférieurs ou des accidents vasculaires cérébraux, pour la cicatrisation des plaies chroniques incluant les ulcères de jambe à prédominance veineuse ou mixtes et le pied diabétique, pour le traitement ou la prévention des crises hémorroïdaires, pour le traitement ou la prévention des escarres et pour le traitement de la sclérose en plaque.

8. Composé selon l'une quelconque des revendications 1 ou 2 utile pour la prévention ou le traitement de la maladie veineuse chronique.

## Claims

1. Compound of formula (I): 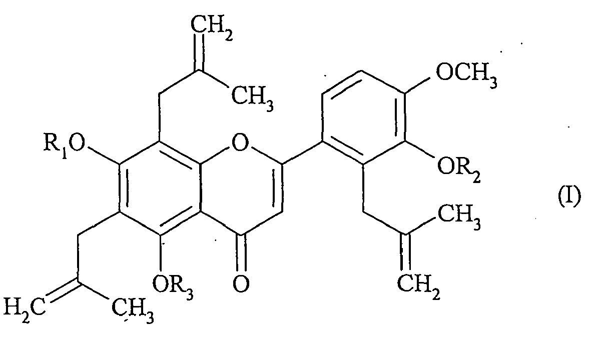 wherein R₁, R₂ and R₃, which may be the same or different, each represent a hydrogen atom or the group of formula (A):

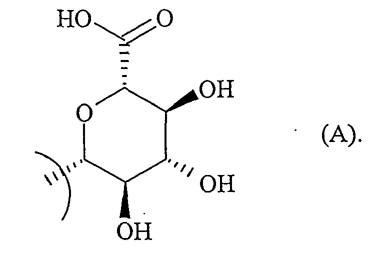

2. Compound of formula (I) according to claim 1, selected from:
- 6,8,2'-tris(isobut-2-en-1-yl)diosmetin,
- (5-hydroxy-2-[3-hydroxy-4-methoxy-2-(isobut-2-en-1-yl)phenyl]-6,8-bis(isobut-2-en-1-yl)-4-oxo-4*H*-chromen-7-yl)-beta-D-glucuronic acid, and
- 3-[5,7-dihydroxy-6,8-bis(isobut-2-en-1-yl)-4-oxo-4*H*-chromen-2-yl]-6-methoxy-2-(isobut-2-en-1 -yl)phenyl-beta-D-glucuronic acid.

3. Process for the synthesis of compounds of formula (I) according to claim 1, starting from diosmetin of formula (II): 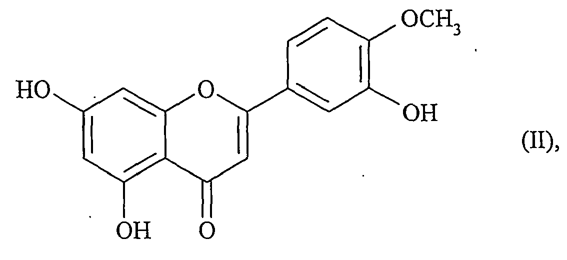 which is reacted with methallyl bromide to yield the compound of formula (III): 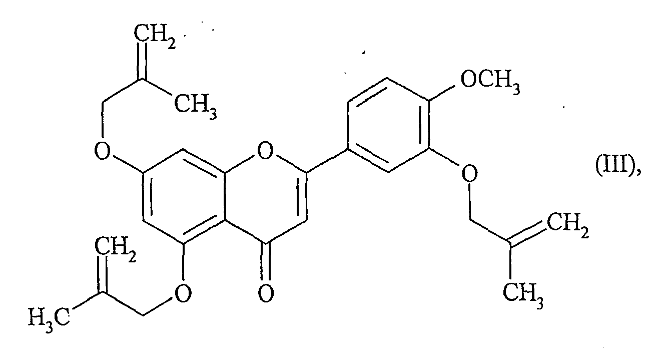 which is heated to yield the compound of formula (Ia), a particular case of the compounds of formula (I) wherein R₁, R₂ and R₃ each represent a hydrogen atom: 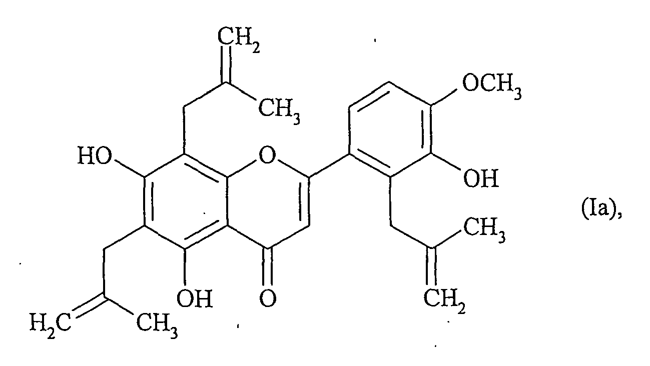 which, when it is desired to obtain other compounds of formula (I), is reacted with the compound of formula (IV): 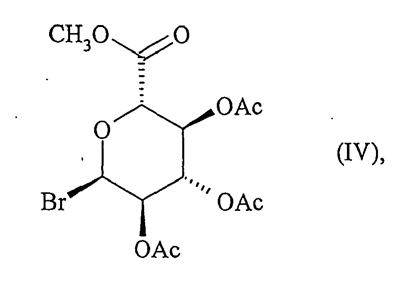 wherein Ac represents the acetyl group,
to yield, after deprotection of the acid function and the alcohol functions of the group (A) as defined in claim 1, compounds of formula (I) wherein at least one of R₁, R₂ and R₃ is other than H.

4. Pharmaceutical composition comprising as active ingredient a compound of formula (I) according to either claim 1 or claim 2, in combination with one or more pharmaceutically acceptable, non-toxic, inert carriers or excipients.

5. Use of a compounds according to either claim 1 or claim 2 in the manufacture of medicaments which are useful in the prevention or treatment of venous diseases, in the prevention or treatment of post-thrombotic syndrome, vascular complications associated with diabetes, hypertension, atherosclerosis, inflammation, metabolic syndrome associated with obesity, vascular complications associated with obesity, angina pectoris, arteritis of the lower limbs or cerebral vascular accidents, in the healing of chronic wounds including mainly venous or mixed leg ulcers and diabetic foot, in the treatment or prevention of haemorrhoid attacks, in the treatment or prevention of pressure ulcers and in the treatment of multiple sclerosis.

6. Use of a compound according to either claim 1 or claim 2 in the manufacture of medicaments which are useful in the prevention or treatment of chronic venous disease.

7. Compound according to either claim 1 or claim 2 for use in the prevention or treatment of venous diseases, in the prevention or treatment of post-thrombotic syndrome, vascular complications associated with diabetes, hypertension, atherosclerosis, inflammation, metabolic syndrome associated with obesity, vascular complications associated with obesity, angina pectoris, arteritis of the lower limbs or cerebral vascular accidents, in the healing of chronic wounds including mainly venous or mixed leg ulcers and diabetic foot, in the treatment or prevention of haemorrhoid attacks, in the treatment or prevention of pressure ulcers and in the treatment of multiple sclerosis.

8. Compound according to either claim 1 or claim 2 for use in the prevention or treatment of chronic venous disease.

## Patentansprüche

1. Verbindung der Formel (I): 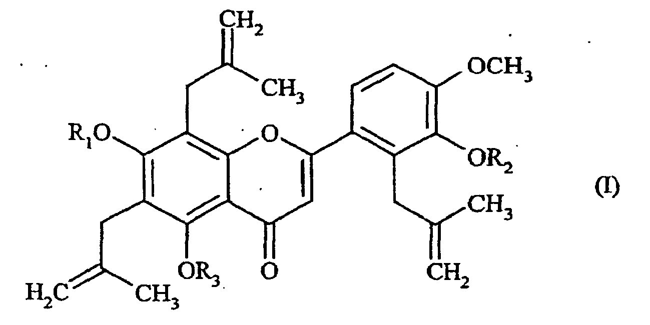 in der R₁, R₂ und R₃, die identisch oder verschieden sind, jeweils ein Wasserstoffatom oder die Gruppe der Formel (A) bedeuten:

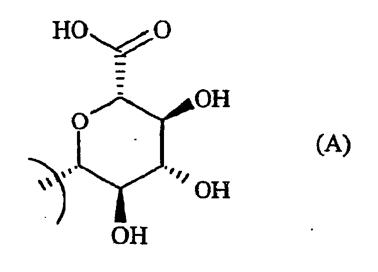

2. Verbindung der Formel (I) nach Anspruch 1, ausgewählt aus:
- 6,8,2'-Tris(isobut-2-en-1-yl)-diosmetin,
- (5-Hydroxy-2-[3-hydroxy-4-methoxy-2-(isobut-2-en-1-yl)-phenyl]-6,8-bis(isobut-2-en-1-yl)-4-oxo-4*H*-chromen-7-yl)-beta-D-glucuronsäure und
- 3-[5,7-Dihydroxy-6,8-bis(isobut-2-en-1-yl)-4-oxo-4*H*-chromen-2-yl]-6-methoxy-2-(isobut-2-en-1-yl)-phenyl-beta-D-glucuronsäure.

3. Verfahren zur Synthese der Verbindungen der Formel (I) nach Anspruch 1, ausgehend von Diosmetin der Formel (II): 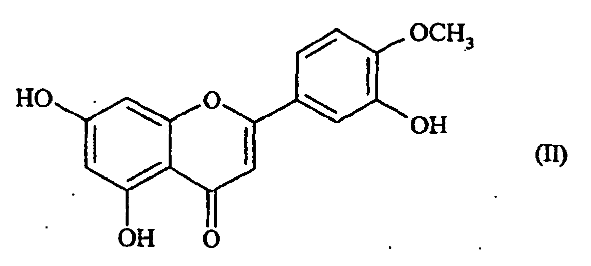 welches man mit Methallylbromid umsetzt zur Bildung der Verbindung der Formel (III): 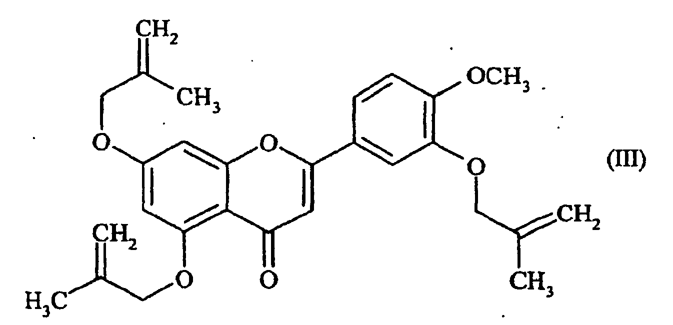 welche man erhitzt zur Bildung der Verbindung der Formel (Ia), einem Sonderfall der Verbindungen der Formel (I), worin R₁, R₂ und R₃ jeweils ein Wasserstoffatom bedeuten: 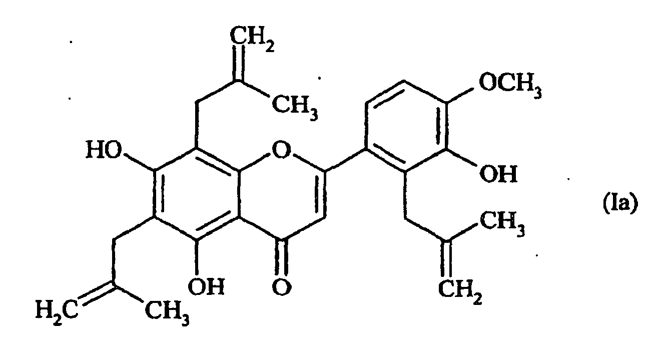 welche man, wenn man andere Verbindungen der Formel (I) herstellen will, mit der Verbindung der Formel (IV) umsetzt: 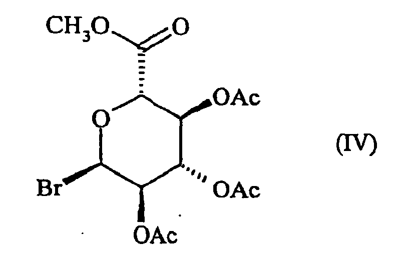 in der Ac die Acetylgruppe bedeutet,
sodass man nach der Abspaltung der Schutzgruppen der Säurefunktion und der Alkoholfunktionen der Gruppe (A), wie sie in Anspruch 1 definiert sind, die Verbindungen der Formel (I) erhält, worin mindestens eine der Gruppen R₁, R₂ und R₃ von H verschieden ist.

4. Pharmazeutische Zubereitung enthaltend als Wirkstoff eine Verbindung der Formel (I) nach einem der Ansprüche 1 oder 2, in Kombination mit einem oder mehreren inerten, nichttoxischen, pharmazeutisch annehmbaren Hilfsstoffen oder Trägermaterialien.

5. Verwendung einer Verbindung nach einem der Ansprüche 1 oder 2 zur Herstellung von Arzneimitteln, die nützlich sind zur Vorbeugung oder Behandlung von Venenerkrankungen, zur Vorbeugung oder Behandlung des post-thrombotischen Syndroms, von mit dem Diabetes verknüpften Gefäßkomplikationen, der Hypertension, der Atherosklerose, von Entzündungen, des mit der Fettsucht verknüpften Stoffwechselsyndroms, mit der Fettsucht verknüpften Gefäßkomplikationen, Angina pectoris, Artheritis der unteren Gliedmaßen oder Gehirngefäßvorfälle (Schlaganfälle), zur Vernarbung von chronischen Wunden einschließlich Beingeschwüren mit venöser oder gemischter Prädominanz und des diabetischen Fußes, zur Behandlung oder Vorbeugung von hämorrhoidalen Krisen, zur Behandlung oder Vorbeugung von Schorf und zur Behandlung von Multipler Sklerose.

6. Verwendung einer Verbindung nach einem der Ansprüche 1 oder 2 zur Herstellung von Arzneimitteln, die nützlich sind zur Vorbeugung oder Behandlung von chronischen Venenerkrankungen.

7. Verbindung nach einem der Ansprüche 1 oder 2, die nützlich ist zur Vorbeugung oder Behandlung von Venenerkrankungen, zur Vorbeugung oder Behandlung des post-thrombotischen Syndroms, von mit dem Diabetes verknüpften Gefäßkomplikationen, der Hypertension, der Atherosklerose, von Entzündungen, des mit der Fettsucht verknüpften Stoffwechselsyndroms, mit der Fettsucht verknüpften Gefäßkomplikationen, Angina pectoris, Artheritis der unteren Gliedmaßen oder Schlaganfällen, zur Vernarbung von chronischen Wunden einschließlich Geschwüren der Beine mit venöser oder gemischter Prädominanz und dem diabetischen Fuß, zur Behandlung oder Vorbeugung von hämorrhoidalen Krisen, zur Behandlung oder Vorbeugung von Schorf und zur.Behandlung von Multipler Sklerose.

8. Verbindung nach einem der Ansprüche 1 oder 2, nützlich zur Vorbeugung oder Behandlung von chronischen Venenerkrankungen.
